# EUROPÄISCHE PATENTSCHRIFT

(11) **EP 1 744 689 B1**
(45) Veröffentlichungstag und Bekanntmachung des Hinweises auf die Patenterteilung: **05.12.2007**
(21) Anmeldenummer: 05817961.5
(22) Anmeldetag: 19.12.2005
(51) Int. Cl.: A61B 17/58

(54) **KLEINFRAGMENT-HUMERUSKOPFPLATTE**
SMALL FRAGMENT HUMERAL HEAD PLATE
PLAQUE POUR LA TETE DE L'HUMERUS A PETITS FRAGMENTS

(30) Priorität: 13.01.2005 DE 202005000508 U
(43) Veröffentlichungstag der Anmeldung: 24.01.2007
(73) Patentinhaber: Königsee Implantate und Instrumente zur Ostheosynthese GmbH, 07426 Königsee-Aschau (DE)
(72) Erfinder: ORSCHLER, Erich, 96472 Rödental (DE); ORSCHLER, Frank, 07426 Königsee (DE); FINGER, Ulrich, 07318 Saalfeld (DE); EHRHARDT, Arndt, 07426 Dörnfeld (DE)
(74) Vertreter: Kruspig, Volkmar
(86) Internationale Anmeldenummer: PCT/EP2005/013679
(87) Internationale Veröffentlichungsnummer: WO 2006/074792

(56) Entgegenhaltungen:
- WO-A-01/19267
- WO-A-02/096309

## Beschreibung

Die Erfindung betrifft eine Kleinfragment-Humeruskopfplatte zur stabilen Fixierung von Knochenbruchstücken am körpernahen Humeruskopf und -schaft, bestehend aus einem langgestreckten, im Querschnitt bogenförmigen Plattenteil sowie einem sich hieran anschließenden, anatomisch vorgeformten Kopfteil, im Plattenteil und im Kopfteil vorgesehenen Bohrungen mit konischem Gewinde zur Aufnahme von Knochenschrauben, welche jeweils einen Schraubenkopf mit einem komplementären konischen Gewinde besitzen, so dass sich beim Einschrauben eine winkelstabile Lage einstellt, gemäß Oberbegriff des Patentanspruchs 1. Das Dokument WO-A-01/19267 stellt den nächsten Stand der Technik dar.

Osteosyntheseplatten aus Titan oder Implantatstahl, auch in anatomisch vorgeformter Gestalt, gehören zum Stand der Technik.

Derartige Platten werden in ihren Längs- und Querschnittsabmessungen an die jeweiligen notwendigen operativen Gegebenheiten angepasst und besitzen im Kopfteil, aber auch im Plattenteil verschiedene Bohrungen zur Aufnahme von Kortikalis- oder Spongiosaschrauben. Der Durchmesser der Bohrungen ist hierbei an die entsprechenden Schrauben angepasst, wobei es ebenfalls bekannt ist, ein konisches Gewinde in die Bohrungen einzubringen, welches mit einem konischen Gegengewinde eines Kortikalis-Schraubenkopfes korrespondiert, so dass eine eingedrehte Schraube eine winkelstabile Lage einnimmt. Bei derartigen winkelstabilen Schraubverbindungen ist es nicht zwingend notwendig, dass die Platte auf der Knochenoberfläche aufliegt, was den Heilungsprozess beschleunigt.

Bei winkelstabilen Kleinfragment-Humeruskopfplatten besteht die Aufgabe der stabilen Fixierung von Knochenfragmenten am körpernahen Humerus. Insbesondere dann, wenn der Humeruskopf in mehrere Fragmente gespalten ist, weist der Humeruskopf mehrere Bohrungen zur Aufnahme einer entsprechenden Anzahl von Schrauben zur Fixierung auf.

Es hat sich jedoch gezeigt, dass gerade dann, wenn im Kopfteil einer solchen Platte eine Vielzahl von Schraubenlöchern vorhanden ist, die Stabilität der Platte leidet mit der Folge, dass insbesondere im Übergangsbereich zwischen Kopfteil und Plattenteil eine Bruchgefahr besteht.

Aus dem Vorgenannten ist es daher Aufgabe der Erfindung, eine weiterentwickelte Kleinfragment-Humeruskopfplatte zur stabilen Fixierung von Knochenbruchstücken am körpernahen Humeruskopf und -schaft anzugeben, wobei die neuartige Platte es ermöglichen soll, auch eine größere Anzahl von Fragmenten sicher zu fixieren, ohne dass die Gesamtstabilität der Platte in unerwünschter Weise leidet oder die operative Handhabung erschwert ist.

Die Lösung der Aufgabe der Erfindung erfolgt mit einer Kleinfragment-Humeruskopfplatte zur stabilen Fixierung von Knochenbruchstücken am körpernahen Humeruskopf und -schaft gemäß der Merkmalskombination nach Patentanspruch 1, wobei die Unteransprüche mindestens zweckmäßige Ausgestaltungen und Weiterbildungen umfassen.

Erfindungsgemäß ist zunächst im Kopfteil eine symmetrisch verteilte Gruppe von Bohrungen ausgebildet, wobei die Symmetrieachse mit der Längsmittelachse der Platte zusammenfällt.

Die ersten, kopfteiläußeren Bohrungen stehen in einem Winkel von etwa 12° bis 17°, vorzugsweise 15° zueinander.

Die zweiten, kopfteilinneren Bohrungen der symmetrischen Bohrungsgruppe sind in einem Winkel zueinander orientiert, der kleiner als der Winkel ist, unter dem die kopfteiläußeren Bohrungen zueinander stehen. Dieser kleinere Winkel beträgt vorzugsweise 10°.

Die ersten und die zweiten Bohrungen der symmetrisch verteilten Gruppe weisen bezogen auf die Senkrechte der Plattenlängsachse eine Schrägstellung hin gerichtet zum Kopfteiläußeren auf.

Erfindungsgemäß ist weiterhin vorzugsweise auf der Längsmittelachse in einem vorgegebenen Abstand vom proximalen Ende der Platte eine Zusatzbohrung ausgebildet, deren Schrägstellung größer als diejenige der ersten und zweiten Bohrungen ist.

Mit Hilfe der vorerwähnten Zusatzbohrung kann insbesondere ein oberes Kalottenfragment besser fixiert werden.

Der vorgegebene Abstand der Zusatzbohrung vom proximalen Ende liegt im Bereich zwischen <25mm und >10mm, bei einer beispielhaften Ausführung einer winkelstabilen Humeruskopfplatte bei etwa 20mm ausgehend vom proximalen Plattenende bzw. Plattenrand.

Die Zusatzbohrung dient der Aufnahme einer winkelstabilen Kopfverriegelungsschraube im Bereich von etwa 3,5mm bis 4mm Durchmesser.

Der Schrägstellungswinkel der Zusatzbohrung liegt bezogen auf die Senkrechte zur Plattenlängsachse bei im Wesentlichen 30° und 40°.

Der Schrägstellungswinkel der Zusatzbohrung lässt sich auch bezogen auf die Längsmittelachse im Bereich zwischen 55° und 75°, vorzugsweise bei 60° bis 70° liegend, definieren.

Die seitliche Winkelstellung der Zusatzbohrung liegt bezogen auf die Plattenoberfläche im Bereich zwischen +10° und -10°, vorzugsweise bei 0°.

Die im Plattenteil vorgesehenen Bohrungen liegen bevorzugt auf der Längsmittelachse. Mindestens einige dieser Bohrungen sind als so genanntes Kombiloch mit einem konischen Gewinde sowie einer sphärischen Versenkung zur Aufnahme von entweder winkelstabilen, aber auch Schrauben mit sphärischem Kopf ausgeführt.

Bevorzugt im Übergangsrandbereich zwischen Kopfteil und Plattenteil ist mindestens eine Bohrung kleineren Durchmessers und Unterschnitt vorgesehen. Diese Bohrung oder derartig ausgeführte mehrere Bohrungen dienen zur Aufnahme von Nadel und Faden dann, wenn die Platte auf dem Knochen liegend vernäht wird.

In einer bevorzugten Ausgestaltung der Erfindung ist im Kopfteil eine weitere Gewindebohrung mit kleinerem Durchmesser sowie eine Führungsbohrung vorgesehen, um einen massiven Zielblock zeitweise zu fixieren.

Der Zielblock weist erste und zweite Bohrungen sowie eine Zusatzbohrung komplementär zu denjenigen im Kopfteil auf, so dass der Zielblock als Bohrschablone und zur Schraubenführung einsetzbar ist.

Das Kopfteil weist im Verbund mit den eingedrehten Schrauben leicht nachgiebige, federnde Eigenschaften auf, so dass eine gewisse Dynamik gegeben ist, was den Heilungsprozess fördert.

Durch den Zielblock und dessen Nutzung als Bohrschablone oder zur Bohrerführung kann die Platte selbst aus einem dünneren Material gefertigt werden, was operative Vorteile bietet.

Im Weiteren besitzt die Platte an sich bekannte Bohrungen zum Fixieren eines Kirschner-Drahts, und zwar am Anfang und am Plattenende.

Die Erfindung soll nachstehend anhand eines Ausführungsbeispiels sowie unter Zuhilfenahme einer Figur näher erläutert werden.

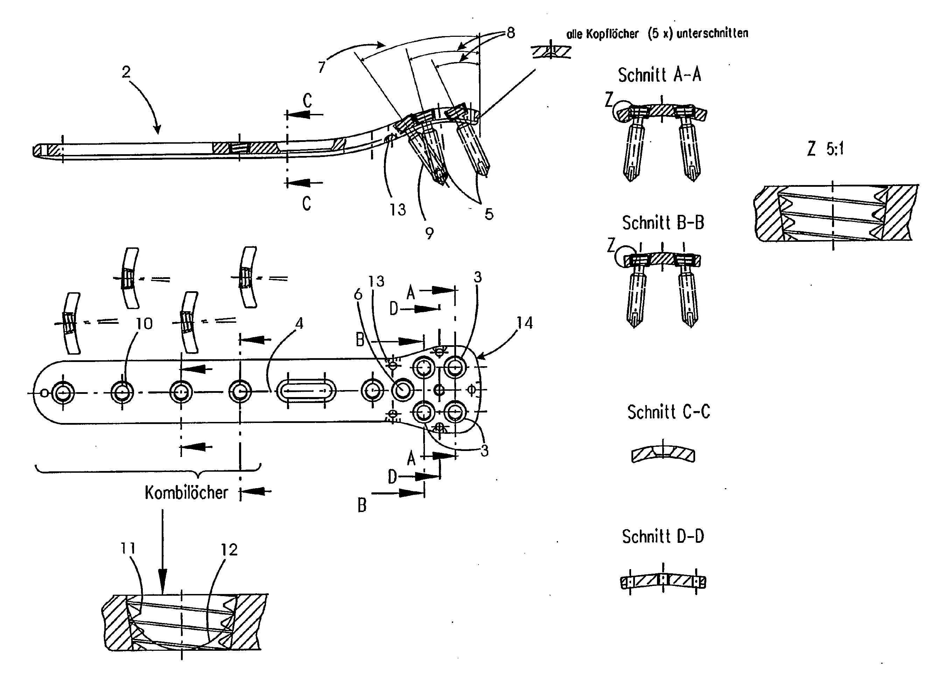

Die Figur zeigt hierbei eine Draufsicht auf eine winkelstabile Humerusplatte gemäß Ausführungsbeispiel. Weiterhin zeigt die Figur eine Seitenansicht mit Teilschnitt sowie verschiedene Schnitte längs der Linien gemäß Draufsicht und Seitenansicht zur Verdeutlichung insbesondere der Winkellagen der Bohrungen.

Ebenfalls ist der Figur die prinzipielle Ausführungsform der Kombilöcher im Plattenlängsteil entnehmbar.

Die Humeruskopfplatte gemäß Ausführungsbeispiel besteht aus einem Kopfteil 1 und einem Plattenteil 2 in langgestreckter Form.

Im Kopfteil ist eine symmetrisch verteilte Gruppe von Bohrungen 3 ausgebildet, wobei die Symmetrieachse mit der Längsmittelachse 4 zusammenfällt.

Die ersten kopfteiläußeren Bohrungen sind so ausgeführt, dass darin aufgenommene Schrauben in einem Winkel von 12° bis 17°, vorzugsweise 15° zueinander orientiert sind. Verwiesen sei hier auf die Darstellung gemäß Schnitt A-A.

Die zweiten, kopfteilinneren Bohrungen sind in einem Winkel kleiner als die kopfteiläußeren Bohrungen, vorzugsweise 10° zueinander orientiert, wie dies die Darstellung Schnitt B-B verdeutlicht.

Die ersten und die zweiten Bohrungen weisen bezogen auf die Senkrechte der Plattenlängsachse 4 ein Schrägstellung zum Kopfteiläußeren auf. Diese Schrägstellung ist durch die Orientierung der Schrauben 5 erkennbar.

Weiterhin vorzugsweise auf der Längsmittelachse 4 liegend und in einem vorgegebenen Abstand vom proximalen Ende der Platte befindlich, ist eine Zusatzbohrung 6 ausgebildet, deren Schrägstellungswinkel 7 vom Schrägstellungswinkel 8 der ersten und zweiten Bohrungen abweicht und insbesondere größer als der Schrägstellungswinkel der ersten und zweiten Bohrungen ist.

Der vorgegebene Abstand der Zusatzbohrung 6 vom proximalen Plattenende ist kleiner als 25mm vorgegeben.

Die Zusatzbohrung 6 dient der Aufnahme einer winkelstabilen Kopfverriegelungsschraube 9 analog der Schrauben für die ersten und zweiten Bohrungen.

Wie aus der Figur ersichtlich, liegt der Schrägstellungswinkel für die Zusatzbohrung 6 bezogen auf die Senkrechte zur Plattenlängsachse bei im wesentlichen 30° bis 45°, beim Beispiel konkret bei 35°.

Der entsprechende Schrägstellungswinkel 8 für die übrigen Bohrungen im Kopfteil sind der Bemaßung gemäß Figur entnehmbar.

Die im Plattenteil 2 vorgesehenen Bohrungen 10, die ebenfalls auf der Längsmittelachse 4 liegen, sind zumindest teilweise als Kombilöcher ausgebildet. Dies bedeutet, dass diese Bohrungen sowohl ein konisches Gewinde 11 als auch eine tiefreichende sphärische Versenkung 12 besitzen, so dass entweder eine winkelstabile oder aber auch eine Schraube mit sphärischem Kopf verwendbar ist.

Bevorzugt im Übergangsrandbereich zwischen Kopfteil 1 und Plattenteil 2 ist mindestens eine weitere Bohrung 13 mit kleinerem Durchmesser und Unterschnitt vorgesehen. Diese Bohrung dient der Aufnahme von Nadel und Faden.

Im Kopfteil 1 ist eine weitere Gewindebohrung 14 vorgesehen, um einen massiven Zielblock (nicht gezeigt) zeitweise auf dem Kopfteil 1 zu fixieren. Der Zielblock besitzt erste und zweite Bohrungen sowie eine Zusatzbohrung komplementär zu denjenigen im Kopfteil, so dass der Zielblock als Bohrschablone, d.h. zur Führung des Bohrers, aber auch als Hilfe zum Einführen der Schrauben verwendet werden kann. Durch diese Maßnahme ist es möglich, die Platte selbst in geringerer Dicke auszuführen.

Das Kopfteil 1 besitzt in Verbindung mit den verwendeten Schrauben nachgiebige, leicht federnde Eigenschaften, so dass sich eine gewisse Dynamik einstellt, was den Heilungsprozess nach einer Fraktur fördert.

## Patentansprüche

1. Kleinfragment-Humeruskopfplatte zur stabilen Fixierung von Knochenbruchstücken am körpernahen Humeruskopf und -schaft, bestehend aus einem langgestreckten, im Querschnitt bogenförmigen Plattenteil (2) sowie einem sich hieran anschließenden, anatomisch vorgeformten Kopfteil (1), im Plattenteil (2) und im Kopfteil (1) vorgesehenen Bohrungen (3) mit konischem Gewinde zur Aufnahme von Knochenschrauben, welche jeweils einen Schraubenkopf mit einem komplementären konischen Gewinde besitzen, so dass sich beim Einschrauben eine winkelstabile Lage einstellt,
**dadurch gekennzeichnet, dass**
im Kopfteil (1) eine symmetrisch verteilte Gruppe von Bohrungen (3) ausgebildet ist, wobei die Symmetrieachse mit der Längsmittelachse (4) der Platte zusammenfällt,
die ersten, kopfteiläußeren Bohrungen (3) in einem Winkel von etwa 12° bis 17°, vorzugsweise 15° zueinander orientiert sind,
die zweiten, kopfteilinneren Bohrungen (3) in einem Winkel kleiner als die kopfteiläußeren Bohrungen (3), vorzugsweise in etwa bei 10° zueinander orientiert sind,
die ersten und zweiten Bohrungen (3), bezogen auf die Senkrechte der Plattenlängsachse (4) eine Schrägstellung zum Kopfteiläußeren aufweisen und
weiterhin vorzugsweise auf der Längsmittelachse (4) in einem vorgegebenen Abstand vom proximalen Ende der Platte eine Zusatzbohrung (6) ausgebildet ist, deren Schrägstellungswinkel (7) größer als derjenige der ersten und zweiten Bohrungen (3) ist.

2. Platte nach Anspruch 1,
**dadurch gekennzeichnet, dass**
der vorgegebene Abstand der Zusatzbohrung vom proximalen Plattenende im Bereich zwischen <25mm und >10mm liegt.

3. Platte nach Anspruch 2,
**dadurch gekennzeichnet, dass**
die Zusatzbohrung zur Aufnahme einer winkelstabilen Kopfverriegelungsschraube im Bereich zwischen 3,5mm und 4mm Durchmessern ausgebildet ist.

4. Platte nach einem der vorangegangenen Ansprüche,
**dadurch gekennzeichnet, dass**
der Schrägstellungswinkel der Zusatzbohrung bezogen auf die Senkrechte zur Plattenlängsachse bei im Wesentlichen 30° bis 40° liegt.

5. Platte nach einem der Ansprüche 1 bis 3,
**dadurch gekennzeichnet, dass**
der Schrägstellungswinkel der Zusatzbohrung bezogen auf die Längsmittelachse im Bereich zwischen 55° 75°, vorzugsweise zwischen 60° und 70° liegt.

6. Platte nach einem der vorangegangenen Ansprüche,
**dadurch gekennzeichnet, dass**
die seitliche Winkelstellung der Zusatzbohrung bezogen auf die Plattenoberfläche im Bereich zwischen +10° und -10°, vorzugsweise bei 0° liegt.

7. Platte nach einem der vorangegangenen Ansprüche,
**dadurch gekennzeichnet, dass**
die im Plattenteil vorgesehenen Bohrungen auf der Längsmittelachse liegen und mindestens einige dieser Bohrungen als Kombiloch mit einem konischen Gewinde sowie einer sphärischen Versenkung zur Aufnahme von entweder winkelstabilen oder aber Schrauben mit sphärischem Kopf ausgeführt sind.

8. Platte nach einem der vorangegangenen Ansprüche,
**dadurch gekennzeichnet, dass**
bevorzugt im Übergangsrandbereich zwischen Kopfteil und Plattenteil mindestens eine Bohrung kleineren Durchmessers und Unterschnitt vorgesehen ist.

9. Platte nach einem der vorangegangenen Ansprüche,
**dadurch gekennzeichnet, dass**
im Kopfteil eine weitere Gewindebohrung sowie mindestens eine Führungsbohrung vorgesehen ist, um einen massiven Zielblock zeitweise zu fixieren, wobei im Zielblock erste und zweite Bohrungen sowie eine Zusatzbohrung komplementär zu denjenigen im Kopfteil vorhanden sind, so dass der Zielblock als Bohrschablone und zur Schraubenführung einsetzbar ist.

10. Platte nach einem der vorangegangenen Ansprüche,
**dadurch gekennzeichnet, dass**
das Kopfteil im Verbund mit den Schrauben leicht nachgiebige, federnde Eigenschaften aufweist.

## Claims

1. Small-fragment humeral head plate which is used to fix, in a stable manner, bone fracture fragments to the humeral head and shaft near to the body, comprising an elongated plate part (2), which has an arched cross-section, and an anatomically preformed head part (1) adjacent thereto, bores (3) which are provided in the plate part (2) and in the head part (1), said bores (3) having a conical thread used to receive bone screws which comprise, respectively, a screw head with a complementary conical thread, such that an angularly stable position is produced upon screwing,
**characterized in that**
a symmetrically distributed group of bores (3) are embodied in the head part (1), wherein the axis of symmetry coincides with the longitudinal central axis (4) of the plate,
the first, external bores (3) on the head part are oriented in relation to each other at an angle of approximately 12° to 17°, preferably 15°,
the second, internal bores (3) on the head part are oriented in relation to each other at an angle which is smaller than that of the external bores (3) on the head part, preferably at approximately 10°,
the first and second bores (3) have an inclined position towards the head part exterior in relation to the perpendicular of the longitudinal axis (4) of the plate, and
further an additional bore (6) is preferably embodied on the longitudinal central axis (4) at a predetermined distance in relation to the proximal end of the plate, the inclined position angle (7) of which is greater than that of the first and second bores (3).

2. Plate according to claim 1,
**characterized in that**
the predetermined distance of the additional bore in relation to the proximal end of the plate is within the range between <25mm and >10mm.

3. Plate according to claim 2,
**characterized in that**
the additional bore is embodied in the diameter range between 3.5mm and 4mm for receiving an angularly stable head attachment screw.

4. Plate according to one of the preceding claims,
**characterized in that**
the inclined position angle of the additional bore in relation to the perpendicular to the longitudinal axis of the plate is substantially at 30° to 40°.

5. Plate according to one of claims 1 to 3,
**characterized in that**
the inclined position angle of the additional bore in relation to the longitudinal central axis is in the range between 55° and 75°, preferably between 60° to 70°.

6. Plate according to one of the preceding claims,
**characterized in that**
the lateral angle position of the additional bore in relation to the plate upper surface is in the range between +10° and -10°, preferably at 0°.

7. Plate according to one of the preceding claims,
**characterized in that**
the bores provided in the plate part are positioned on the longitudinal central axis and that at least some of these bores are embodied as a combined hole, comprising a conical thread and a spherical countersink for receiving either angularly stable screws or screws having a spherical head.

8. Plate according to one of the preceding claims,
**characterized in that**
at least one smaller diameter bore including a small lateral groove is preferably provided in the transitional edge region between the head part and the plate part.

9. Plate according to one of the preceding claims,
**characterized in that**
a further threaded bore as well as at least one guide bore are provided in the head part so as to temporarily fix a massive target block, wherein the target block comprises first and second bores as well as an additional bore complementarily to those in the head part, so that the target block can be used as drilling template and screw guide.

10. Plate according to one of the preceding claims,
**characterized in that**
the head part has, in combination with the screws, slightly yielding, resilient properties.

## Revendications

1. Plaque destinée à des petits fragments de tête d'humérus pour la fixation stable de morceaux osseux sur la tête et la tige de l'humérus proches du corps, constituée par une portion de plaque allongée (2) de section transversale en forme d'arc ainsi que par une portion de tête (1) anatomiquement préformée qui s'y raccorde, par des perçages (3) prévus dans la portion de plaque (2) et dans la portion de tête (1) et présentant des taraudages coniques pour recevoir des vis à os qui possèdent chacune une tête de vis munie d'un filetage conique complémentaire, de sorte qu'une position angulaire stable s'établit lors du vissage,
**caractérisée en ce que**
un groupe de perçages (3) répartis symétriquement sont ménagés dans la portion de tête (1), l'axe de symétrie coïncidant avec l'axe longitudinal médian (4) de la plaque,
les premiers perçages (3) à l'extérieur dans la portion de tête sont orientés les uns par rapport aux autres sous un angle d'environ 12° à 17°, de préférence de 15°,
les seconds perçages (3) à l'intérieur dans la portion de tête sont orientés les uns par rapport aux autres sous un angle inférieur à celui des perçages (3) à l'extérieur dans la portion de tête, de préférence d'environ 10°,
par rapport à la perpendiculaire à l'axe longitudinal (4) de la plaque, les premiers et les seconds perçages (3) présentent une position oblique par rapport à l'extérieur de la portion de tête, et de plus un perçage supplémentaire (6) est ménagé de préférence sur l'axe longitudinal médian (4), à une distance prédéterminée de l'extrémité proximale de la plaque, perçage dont l'angle de position oblique (7) est supérieur à celui des premiers et des seconds perçages (3).

2. Plaque selon la revendication 1,
**caractérisée en ce que**
la distance prédéterminée du perçage supplémentaire vis-à-vis de l'extrémité proximale de la plaque est dans la plage entre < 25 mm et > 10 mm.

3. Plaque selon la revendication 2,
**caractérisée en ce que**
le perçage supplémentaire est réalisé pour recevoir une vis de verrouillage de tête à angle stable et d'un diamètre dans la plage entre 3,5 mm et 4 mm.

4. Plaque selon l'une des revendications précédentes,
**caractérisée en ce que**
l'angle de position oblique du perçage supplémentaire par rapport à la perpendiculaire à l'axe longitudinal de la plaque est sensiblement de 30° à 40°.

5. Plaque selon l'une des revendications 1 à 3,
**caractérisée en ce que**
l'angle de position oblique du perçage supplémentaire par rapport à l'axe longitudinal médian est dans la plage entre 55° et 75°, de préférence entre 60° et 70°.

6. Plaque selon l'une des revendications précédentes,
**caractérisée en ce que**
la position angulaire latérale du perçage supplémentaire par rapport à la surface de plaque est dans la plage entre +10° et -10°, de préférence de 0°.

7. Plaque selon l'une des revendications précédentes,
**caractérisée en ce que**
les perçages prévus dans la portion de plaque se trouvent sur l'axe longitudinal médian et au moins quelques-uns de ces perçages sont réalisés sous forme de trou combiné présentant un taraudage conique ainsi qu'un lamage sphérique pour recevoir soit des vis à angle stable soit des vis à tête sphérique.

8. Plaque selon l'une des revendications précédentes,
**caractérisée en ce que**
il est prévu au moins un perçage de petit diamètre et à dépouille de préférence dans la zone marginale de transition entre la portion de tête et la portion de plaque.

9. Plaque selon l'une des revendications précédentes,
**caractérisée en ce que**
dans la portion de tête sont prévus un autre perçage taraudé ainsi qu'au moins un perçage de guidage pour fixer temporairement un bloc cible massif, et dans le bloc cible sont prévus des premiers et des seconds perçages ainsi qu'un perçage supplémentaire de façon complémentaire à ceux dans la portion de tête, de sorte que le bloc cible est utilisable à titre de gabarit de perçage et pour le guidage des vis.

10. Plaque selon l'une des revendications précédentes,
**caractérisée en ce que**
la portion de tête présente, en association avec les vis, des propriétés légèrement souples à effet de ressort.
